# EUROPEAN PATENT SPECIFICATION

(11) **EP 3 384 860 B1**
(45) Date of publication and mention of the grant of the patent: **20.01.2021**
(21) Application number: 18165683.6
(22) Date of filing: 04.04.2018
(51) Int. Cl.: A61B 17/3207

(54) **INTRAVASCULAR CATHETER HAVING AN EXPANDABLE INCISING PORTION AND GRATING TOOL**
INTRAVASKULÄRER KATHETER MIT ERWEITERBAREM SCHNEIDEBEREICH UND REIBWERKZEUG
CATHÉTER INTRAVASCULAIRE PRÉSENTANT UNE PARTIE D'INCISION EXPANSIBLE ET UN OUTIL DE RÂPAGE

(30) Priority: 07.04.2017 US 201715481562
(43) Date of publication of application: 10.10.2018
(73) Proprietor: VentureMed Group, Inc., Plymouth, MN 55441 (US)
(72) Inventor: Pigott, John P., Sylvania, OH 43560 (US)
(74) Representative: Haseltine Lake Kempner LLP

(56) References cited:
- WO-A1-2015/190578
- WO-A1-2015/195606
- WO-A1-2016/210167
- US-A- 5 074 871
- US-A- 5 100 425
- US-A- 5 318 576

## Description

### CROSS-REFERENCE TO RELATED APPLICATIONS

This application is a continuation-in-part of Application No. 15/200,767 filed July 1, 2016, and published as US2016/0310163 A1, which is a continuation-in-part of Application No. 13/613,914 filed September 13, 2012, and published as US2013/0066346, which claims the benefit of United States Provisional Application No. 61/534,018, filed September 13, 2011.

### BACKGROUND OF THE INVENTION

This invention relates in general to intravascular catheters, such as can be used during minimally invasive surgical procedures. In particular, this invention relates to an intravascular catheter having an expandable incising portion.

Atherosclerosis is a chronic condition in which atheromatous plaque accumulates on the inner walls of a blood vessel. As a result, the blood vessel walls can become inflamed and, over time, may harden to form atherosclerotic lesions that cause a narrowing of the vessel lumen. In severe cases, the atherosclerotic lesions can rupture and induce the formation of thrombus (i.e., blood clots), which can prevent blood flow through the narrowed vessel lumen.

There are known procedures and devices for treating or otherwise reducing the risks associated with atherosclerosis, such as angioplasty. However, in some cases it would be desirable to fragment the atherosclerotic lesions. Thus, it would be desirable to provide an intravascular catheter having an expandable portion that can be selectively controlled by a user and adapted to create incisions in atherosclerotic material to facilitate fragmentation of the material during an angioplasty procedure.

There are known procedures and devices for performing atherectomy by abrading atherosclerotic tissue through high speed rotational movement. Such devices include the Diamondback 360® by Cardiovascular Systems, Inc.¹ and the Rotablator™ by Boston Scientific™². These devices are complex, requiring high speed motors and control systems to operate the motors. In some cases, it would be desirable to remove atherosclerotic material by grating or peeling it away through axial movement. Thus, it would be desirable to provide an intravascular catheter having an expandable portion and also having a grating tool surface for removing atherosclerotic material by forward and backward axial movement through the blood vessel.
¹ http://www.csi360.com/products/coronary-diamondback-360-coronary-orbital-atherectomy-system-crowns/
¹ http://www.bostonscientific.com/en-US/products/plaque-modification/rotablator-rotational-atherectomy-system.html

Embolism is a risk sometimes associated with surgical procedures such as the treatment of peripheral artery disease. Plaque fragments, clots, or other particulate can become dislodged and travel through a patent's vascular system and cause damage. As such, embolic protection devices are sometimes placed in the patient's vascular system during surgical procedures in order to catch and remove emboli that may form or become dislodged. Use of such devices typically requires the selection, insertion, positioning, and removal of a separate device. Proper design, inventory maintenance, selection, placement, and removal can be difficult, particularly in light of varying patient anatomy, conditions, and treatments being performed. Further, placed devices can be cumbersome and difficult to work around. Thus, it would be desirable to provide an intravascular catheter having an expandable portion and also having an integrated embolic protection device.

US5074871 discloses a catheter atherotome and method for its use for performing partial atherectomy in an artery and thereby enlarging the lumen effectively available for blood flow through the artery. An expansible cutter head at the distal end of a catheter includes several elongate flexible members mounted in a parallel array and spaced angularly apart from one another about the associated ends of two concentric members of the catheter in such a way that longitudinal and rotary relative movement of the members of the catheter selectively either bows the flexible members arcuately outwardly into a cutting position or draws them into alignment parallel with the catheter.

US5100425 discloses an expandable transluminal atherectomy catheter having forwardly directed cutting means (24) on the end of a catheter (22) which can be introduced into the artery into the proximity of the stenosis along a guide wire (32). During insertion the cutting means (24) is collapsed by an outer sleeve (40) which can be retracted after insertion to permit the cutting means to expand to fill the artery.

WO2016210167 discloses a tissue-removing catheter which includes a drive shaft (12) and a tissue- removing head (14). The tissue-removing head is at the distal end of the drive shaft and is configured to rotate about a longitudinal axis of the drive shaft.

WO 2015/195606 A1 discloses an intravascular catheter device comprising an expandable portion with incising members on it.

### SUMMARY OF THE INVENTION

The invention is defined by the independent claim and preferred embodiments are listed in the dependent claims.

This invention relates to an intravascular catheter device for use during a surgical procedure. The catheter device includes a catheter tube having an expandable portion with a plurality of struts each defining an outer surface. The expandable portion is operable between a closed position, wherein the expandable portion has a first diameter, and an opened position, wherein the expandable portion has a second diameter that is larger than the first diameter.

Grating tools are integrated with the struts. In exemplary embodiments, the grating tools may comprise slightly or partially raised apertures, which may further comprise a sharpened portion. The grating tools may be configured to grate or peel away atherosclerotic material as the expandable portion is moved axially forwards and backwards through the blood vessel. An incising element is also provided on the outer surface of one or more of the struts. The incising element may be configured to fragment the atherosclerotic plaque.

An embolic protection device is integrated with the expandable portion. In exemplary embodiments, the embolic protection device is a mesh or film placed over a portion of the struts that is configured and positioned to catch the atherosclerotic material being grated or peeled away.

Various aspects of this invention will become apparent to those skilled in the art from the following detailed description of the preferred embodiments, when read in light of the accompanying drawings.

### BRIEF DESCRIPTION OF THE DRAWINGS

Fig. 1 is a plan view of a catheter device that includes a handle assembly and a catheter tube having an expandable incising portion, in accordance with a first embodiment of this invention.
Fig. 2 is a cross-sectional side view of the handle assembly taken along section line 2-2 shown in Fig. 1 when the catheter device is in a first operating mode.
Fig. 3 is an enlarged cross-sectional side view of the catheter tube taken along section line 3-3 shown in Fig. 1 illustrating the expandable incising portion disposed within a blood vessel.
Fig. 4 is a cross-sectional end view of the expandable incising portion taken along section line 4-4 shown in Fig. 3.
Fig. 5 is a cross-sectional side view of the handle assembly taken along section line 2-2 shown in Fig. 1 when the catheter device is in a second operating mode.
Fig. 6 is an enlarged cross-sectional side view of the catheter tube taken along section line 3-3 shown in Fig. 1 illustrating the expandable incising portion in an opened position.
Fig. 7 is a cross-sectional end view of the expandable incising portion taken along section line 7-7 shown in Fig. 6.
Fig. 8 is an enlarged side view of a catheter tube having an expandable incising portion, in accordance with a second embodiment of this invention.
Fig. 9 is a side view of the catheter tube shown in Fig. 8 illustrating the expandable incising portion in an opened position.
Fig. 10 is a cross-sectional end view of the expandable incising portion taken along section line 10-10 shown in Fig. 9.
Fig. 11 is an enlarged side view of a catheter tube having an expandable incising portion.
Fig. 12 is a side view of the catheter tube shown in Fig. 11 illustrating the expandable incising portion in an opened position.
Fig. 13 is an end view of the catheter tube as shown in Fig. 12.
Fig. 14 is an enlarged side view of a catheter tube having an expandable incising portion.
Fig. 15 is a side view of the catheter tube shown in Fig. 14 illustrating the expandable incising portion in an opened position.
Fig. 16 is an end view of the catheter tube as shown in Fig. 15.
Fig. 17 is a side view of the device shown in Fig. 8 with an exemplary embolic protection device installed thereon.
Fig. 18 is a side view of the device shown in Fig. 17 with the expandable incising portion in the opened position.
Fig. 19 is a side view of the device shown in Fig. 11 with the exemplary embolic protection device installed thereon.
Fig. 20 is a side view of the device shown in Fig. 19 with the expandable incising portion in the opened position.
Fig. 21 is a side view of the device shown in Fig. 11 with another exemplary embolic protection device installed thereon.
Fig. 22 is a side view of the device shown in Fig. 21 with the expandable incising portion in the opened position.
Fig. 23 is a side view of another exemplary embodiment of the device of Fig. 8.
Fig. 24 is a side view of the device of Fig. 23 illustrated in an opened position.
Fig. 25 is a side view of another exemplary embodiment of the device of Fig. 11.
Fig. 26 is a side view of the device of Fig. 25 illustrated in an opened position.
Fig. 27 is a side view of another exemplary embodiment of the device of Fig. 14.
Fig. 28 is a side view of the device of Fig. 27 illustrated in an opened position.
Fig. 29 is a side view of an embodiment according to the invention of the device of Fig. 8.
Fig. 30 is a side view of the device of Fig. 29.

### DETAILED DESCRIPTION OF THE PREFERRED EMBODIMENTS

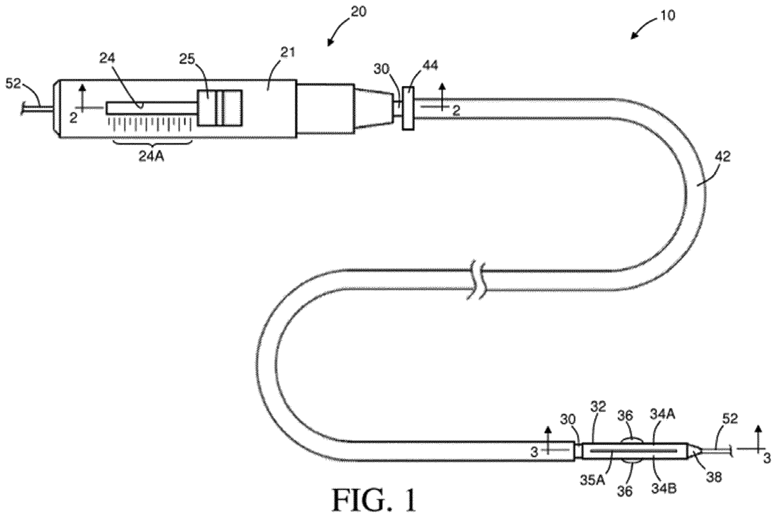

Referring now to the drawings, there is illustrated in Fig. 1 a catheter device, indicated generally at 10, in accordance with this invention. The illustrated catheter device 10 is configured to treat or reduce the risks associated with atherosclerosis. In general, the catheter device 10 includes an expandable incising portion that can be inserted into a blood vessel and expanded to create incisions in atherosclerotic material that has accumulated on inner walls of the blood vessel. The incisions facilitate the fragmentation of the atherosclerotic material during a subsequent angioplasty or atherectomy procedure. Although the catheter device 10 will be described and illustrated in the context of treating atherosclerosis, it should be appreciated that the catheter device 10 can be used in any desired environment and for any desired purpose.

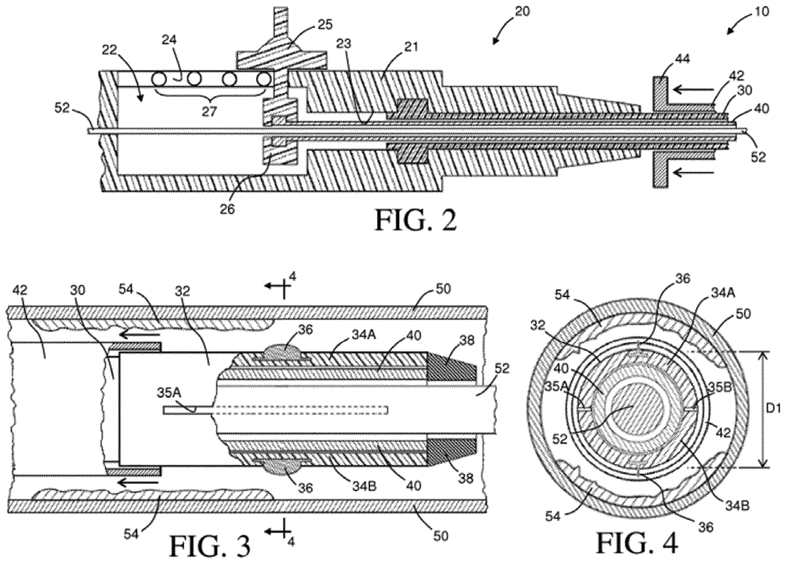

Referring now to Figs. 1 and 2, the illustrated catheter device 10 includes a handle assembly, indicated generally at 20. The illustrated handle assembly 20 includes an elongated, cylindrical handle body 21. The handle body 21 may alternatively have any other shape that is suitable for easy handling by a surgeon. Further, the handle body 21 can be made from any suitably rigid material including, but not limited to, stainless steel or polymers.

As shown in Fig. 2, the illustrated handle body 21 defines an internal chamber 22. A passage 23 extends into an end portion of the handle body 21 for communication with the internal chamber 22. The handle body 21 further includes a slot 24 that extends through a side wall thereof for communication with the internal chamber 22. The illustrated slot 24 may have any length or width as desired. As shown in Fig. 1, an indicator 24A may be provided on the handle body 21 adjacent to the slot 24. For example, the indicator 24A can be a visual scale or any other indicating means, the purpose of which will be explained below.

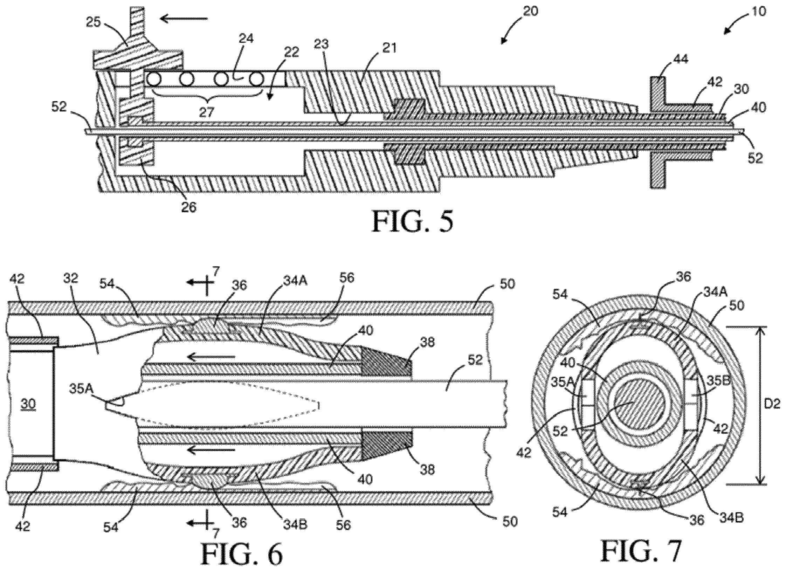

The illustrated handle assembly 20 also includes a control member 25 that is supported on the handle body 21 for sliding movement within the slot 24. For example, the control member 25 is movable between a forward position (shown in Fig. 2), a rearward position (shown in Fig. 5), or any position therebetween, which will be further explained below. As shown in Fig. 2, the illustrated control member 25 includes a base portion 26 that is disposed within the internal chamber 22 of the handle body 21. The base portion 26 may define an outer cross-sectional shape that generally corresponds with a cross-sectional shape of the internal chamber 22, although such is not required. Alternatively, (or in addition), the control member 25 may be movably supported on the handle body 21 by a bearing, a bushing, a guide rail, or any other structural means. In other embodiments, the control member 25 may be supported for rotational movement, pivotal movement, or any other type of movement relative to the handle body 21, the purpose of which will become apparent below. The visual indicator 24A, described above, is configured to identify the relative position of the control member 25 with respect to the handle body 21.

The illustrated handle assembly 20 also includes a locking mechanism 27 that is configured to temporarily secure the control member 25 in a desired position, although such is not required. As shown in Fig. 2, the illustrated locking mechanism 27 includes a plurality of protrusions that are spaced apart from one another along an inner surface of the slot 24. The control member 25 frictionally engages the protrusions to hold the control member 25 in the desired position. Alternatively, the locking mechanism 27 may be a threaded fastener, a pivotal latch, a push-button release, or any other mechanism that is configured to secure the control member 25 in a desired position.

Referring now to Figs. 1 through 3, the illustrated catheter device 10 also includes a catheter tube 30 that extends from the handle assembly 20. The catheter tube 30 is an elongated, flexible member having a proximal end that is secured to the handle assembly 20 and a distal end that extends therefrom. The catheter tube 30 can be made from any biocompatible material including, but not limited to, polyvinyl, polyethylene, nitinol, or stainless steel. Further, the catheter tube 30 can have any outer diameter, length, or wall thickness.

As shown in Fig. 2, the proximal end of the catheter tube 30 is secured to the handle body 21 and communicates with the internal cavity 22 through the passage 23. The catheter tube 30 may be secured to the handle body 21 using a flanged connection, a fused connection, an adhesive, a press-fit connection, a threaded connection, or any other securing means. Alternatively, the catheter tube 30 may be secured to the handle body 21 using a connector or any other type of attachment device.

As shown in Figs. 1 and 3, an expandable portion 32 is provided on the distal end of the catheter tube 30. The illustrated expandable portion 32 is a cylindrical member having a longitudinal axis. The expandable portion 32 can be made from a generally resilient material that is able to flex between various positions, such as polyvinyl, polyethylene, nitinol, or stainless steel. The expandable portion 32 can be secured to the catheter tube 30 in any manner including, but not limited to, a fused connection, an adhesive, a press-fit connection, a threaded connection, or any other securing means. Alternatively, the expandable portion 32 can be integrally formed from the catheter tube 30. Further, the expandable portion 32 can have any outer diameter, length, or wall thickness.

The illustrated expandable portion 32 has a pair of struts 34A and 34B. The illustrated struts 34A and 34B are separated by a pair of longitudinally extending slits 35A and 35B that extend through side walls of the expandable portion 32. As shown in Fig. 4, the slits 35A and 35B are equally spaced apart from one another around the circumference of the expandable portion 32 such that the struts 34A and 34B have the same circumferential widths, although such is not required. The struts 34A and 34B may have any length, circumferential width, or cross-sectional shape as desired.

As shown in Figs. 3 and 4, the illustrated expandable portion 32 also includes a pair of incising elements 36 that are respectively provided along outer surfaces of the struts 34A and 34B. The incising elements 36 can be atherotomes or other incising members having arcuate shaped sharpened edges, for example, that are configured to create incisions in atherosclerotic material as will be explained below. The illustrated incising elements 36 extend parallel with the longitudinal axis of the expandable portion 32 and outwardly in a radial direction therefrom. The incising elements 36 are equally spaced apart from one another around the circumference of the expandable portion 32. The expandable portion 32 may, however, have any number or configuration of incising elements 36 provided around the circumference thereof. Further, the incising elements 36 can have any cross-sectional shape, longitudinal length, or height and can be made from any suitable material including, but not limited to, tempered steel, stainless steel, high carbon steel, or ceramics. The incising elements 36 can be molded with the struts 34A and 34B or may otherwise be secured thereto in any manner such as, for example, using a welded or soldered connection, an adhesive, or any other fastening means.

The distal end of the expandable portion 32 may optionally include a tip member 38. The illustrated tip member 38 has a generally conical shape that facilitates insertion of the catheter tube 30 within a blood vessel 50 (see Figs. 3 and 4) and subsequent travel therethrough. The tip member 38 may, however, have any desired shape. An aperture may axially extend through the tip member 38, the purpose of which will be explained below. The tip member 38 can be integrally formed with the expandable portion 32 or may be secured thereto, such as with an adhesive or the like. Further, the tip member 38 can be made from any biocompatible material including, but not limited to, polyvinyl, polyethylene, nitinol, stainless steel, or polyether block amide.

As shown in Figs. 2 through 4, the illustrated catheter device 10 also includes an inner sleeve 40, although such is not required. The inner sleeve 40 is a flexible, tubular member that is supported for sliding movement within the catheter tube 30, the purpose of which will be explained below. The inner sleeve 40 can be made from any biocompatible material including, but not limited to, polyvinyl, polyethylene, nitinol, stainless steel, or a woven material. Further, the inner sleeve 40 can have any outer diameter, length, or wall thickness. The inner sleeve 40 need not be a tubular member but may alternatively be a solid wire, a braided wire, or the like.

As shown in Fig. 2, a proximal end of the inner sleeve 40 extends from the catheter tube 30 and into the internal chamber 22 of the handle body 21. The proximal end of the inner sleeve 40 is secured to the base portion 26 of the control member 25 for sliding movement therewith, the purpose of which will be explained below. The inner sleeve 40 can be secured to the base portion 26 by a flanged connection, a fused connection, an adhesive, a threaded connection, or any other securing means.

As shown in Fig. 3, the inner sleeve 40 extends through an entire length of the catheter tube 30. A distal end of the inner sleeve 40 that is opposite the handle assembly 20 is secured to the tip member 38, which is in turn secured to the expandable portion 32. The inner sleeve 40 may be secured to the tip member 38 in any manner including, but not limited to, a fused connection, an adhesive, a fastener, or the like.

Referring back to Figs. 1 and 2, the illustrated catheter device 10 also includes a protective sheath 42 that is supported for sliding movement along an outer surface of the catheter tube 30, although such is not required. The protective sheath 42 can be made from any biocompatible material including, but not limited to, polyvinyl, polyethylene, nitinol, or stainless steel. Further, the protective sheath 42 can have any outer diameter, length, or wall thickness. The purpose of the protective sheath 42 will be explained below.

The illustrated protective sheath 42 includes a flange 44 that facilitates sliding movement of the protective sheath 42 relative to the catheter tube 30. The illustrated flange 44 is an annular member that is located at an end of the protective sheath 42 nearest the handle assembly 20. The flange 44 can be integrally formed with the protective sheath 42 or may otherwise be secured thereto in any manner, such as with an adhesive or the like. It should be appreciated that the flange 44 can have any shape or may alternatively be configured in any manner to accomplish the functions described herein and below.

The operation of the catheter device 10 will now be described with reference to Figs. 1 through 7. Referring initially to Figs. 1 through 4, the catheter device 10 is illustrated in a first operating mode. In the first operating mode, the control member 25 on the handle assembly 20 is located in the forward position relative to the handle body 21. The inner sleeve 40 fully extends into the catheter tube 30 such that the expandable portion 32 is in a closed position, as shown in Figs. 3 and 4. In the closed position, the struts 34A and 34B are generally parallel with one another and with the inner sleeve 40. The slits 35A and 35B (illustrated by the dashed lines in Fig. 3) remain in a generally closed configuration. As such, the expandable portion 32 defines an initial diameter D1, which is generally the same diameter as the remaining length of the catheter tube 30. The initial diameter D1 of the expandable portion 32 may, however, be any desired dimension.

When the catheter device 10 is in the first operating mode, the distal end of the catheter tube 30 can be percutaneously inserted into a blood vessel 50, as shown in Figs. 3 and 4. The illustrated catheter tube 30 is then advanced through the blood vessel 50 along a guide wire 52, which extends through the catheter device 10. For example, the guide wire 52 may fully extend through the inner sleeve 40, into the internal chamber 22 of the handle body 21, and exit a rear end of the handle assembly 20 (see Fig. 2). The catheter tube 30 is advanced along the guide wire 52 until the expandable portion 32 is positioned in a narrowed region of the blood vessel 50 caused by atherosclerotic material 54. Alternatively, the catheter tube 30 can be inserted into the blood vessel 50 and guided therethrough by a delivery catheter (not shown) or any other suitable procedure. During insertion and advancement of the catheter tube 30 through the blood vessel 50, the optional protective sheath 42 is preferably positioned over the expandable portion 32, thereby preventing the incising elements 36 from coming into contact with inner walls of the blood vessel 50.

Once the expandable portion 32 is positioned in the narrowed region of the blood vessel 50, the incising elements 36 can be exposed by sliding the protective sheath 42 back from the distal end of the catheter tube 30, as indicated by the direction arrows in Fig. 3. The illustrated protective sheath 42 can be moved in this manner by pulling the flange 44 towards the handle assembly 20, which is indicated by the direction arrows in Fig. 2.

Referring now to Figs. 5 through 7, the catheter device 10 is illustrated in a second operating mode. To achieve the second operating mode, the control member 25 is moved from the forward position to the rearward position, as indicated by the direction arrow in Fig. 5. As the control member 25 is moved to the rearward position, the inner sleeve 40 is drawn within the catheter tube 30 thereby reducing the relative length of the inner sleeve 40 with respect to the catheter tube 30. The distal end of the inner sleeve 40 is attached to the tip member 38, as described above, causing the expandable portion 32 to become axially compressed between the tip member 38 and the distal end of the catheter tube 30. As a result, the struts 34A and 34B bow or expand outwardly in a generally arcuate fashion thereby defining an opened position. In the opened position, the expandable portion 32 defines a second diameter D2 that is larger than the initial diameter D1 when the expandable portion 32 is in the closed position. As shown in Fig. 6, the incising elements 36 are respectively positioned along the radially outer most surfaces of the struts 34A and 34B. Further, the outer most surfaces of the struts 34A and 34B may define a generally flat portion along a length thereof in the opened position, the purpose of which will be explained below, although such is not required. It should be appreciated that the struts 34A and 34B can have any lengths such that the expandable portion 32 can achieve a desired overall second diameter D2 in the opened position.

During operation of the catheter device 10, the second diameter D2 can be increased or decreased by selective movement of the control member 25 between the forward and rearward positions. For example, a larger second diameter D2 can be achieved by moving the control member 25 further towards the rearward position. Conversely, a smaller second diameter D2 can be achieved by moving the control member 25 further towards the forward position. The visual indicator 24A can be used to identify the instantaneous second diameter D2 of the expandable portion 32. Alternatively (or in addition), the struts 34A and 34B may be biased in the opened position so as to automatically expand outwardly to the second diameter D2 when the protective sheath 42 is slid back from the expandable portion 32. As such, sliding movement of the protective sheath 42 relative to the struts 34A and 34B can be used to selectively control the second diameter D2. In this configuration, the inner sleeve 40 and the movable components of the handle assembly 20 may not be necessary.

When the catheter device 10 is in the second operating mode, the expandable portion 32 can be pulled along the guide wire 52 through the narrowed region of the blood vessel 50. This can be accomplished by pulling on the handle assembly 20. In doing so, the incising elements 36 engage the atherosclerotic material 54 and create longitudinal incisions 56 therein. As shown in Figs. 6 and 7, the outer surface area of the arcuate shaped struts 34A and 34B, which is adjacent to the incising element 36, is configured to ride along a surface of the atherosclerotic material 54, thereby limiting the depth of the incisions 56 and preventing the incising members 36 from cutting the walls of the blood vessel 50. The expandable portion 32 can be moved any distance along the guide wire 52 to create incisions 56 having any desired length. After the incisions 56 are made in the atherosclerotic material 54, the catheter device 10 can be returned to the first operating mode (shown in Figs. 1 through 4) by moving the control member 25 to the forward position. In doing so, the expandable portion 32 returns to the closed position. The protective sheath 42 can be slid over the expandable portion 32 and the catheter tube 30 may be removed from the blood vessel 50.

Alternatively, the catheter device 10 can be used to create additional incisions 56 in the atherosclerotic material 54. For example, after the catheter device 10 has been returned to the first operating mode, the expandable portion 32 can be relocated within the narrowed region of the blood vessel 50. The catheter tube 30 can then be rotated within the blood vessel 50 by rotating the handle assembly 20 so as to align the incising elements 36 with other portions of the atherosclerotic material 54. The previous steps can then be repeated any number of times to make multiple passes through the narrowed region of the blood vessel 50 and create additional incisions in the atherosclerotic material 54.

Thus, it should be appreciated that the illustrated catheter device 10 is advantageous in many respects. In one example, the second diameter D2 of the expandable portion 32 can be selectively controlled by operation of the handle assembly 20 or by sliding movement of the protective sheath 42. This enables the catheter device 10 to be adapted for use in blood vessels 50 of different sizes or varying diameters. In another example, the illustrated catheter device 10 can apply varying magnitudes of radial forces to the atherosclerotic material 54 by controlling the amount of force being applied to the control member 25 on the handle assembly 20. This enables the catheter device 10 to generate sufficient radial force to create incisions 56 in atherosclerotic material 54 while reducing the potential for tearing the walls of the blood vessel 50. In yet another example, the catheter device 10 can be used to make any number of passes during a single procedure to make multiple incisions 56 in atherosclerotic material 54 of varying lengths and shapes.

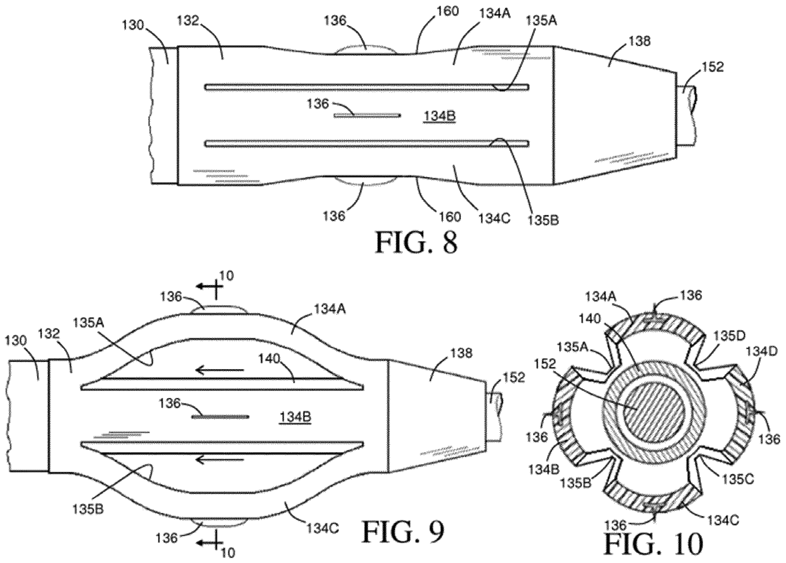

Referring now to Figs. 8 through 10, there is illustrated a catheter tube 130 having an expandable portion 132, in accordance with a second embodiment of this invention. The catheter tube 130 and the expandable portion 132 may include any structural features as described and illustrated above in the previous embodiment, although such is not required. Similar features have been numbered with common reference numerals but have been increased by 100 (i.e., 110, 120, 130, etc.). It should be appreciated that similar features are structured similarly, operate similarly, and/or have the same function unless otherwise indicated by the drawings or this specification.

For example, the catheter tube 130 may extend from a handle assembly (not shown) as described above in the first embodiment. The expandable portion 132 is provided on a distal end of the catheter tube 130 and may include a tip member 138. The catheter tube 130 may also include an inner sleeve 140 and a protective sheath (not shown), which is also described above in the first embodiment.

In the illustrated embodiment, however, the expandable portion 132 includes four struts 134A, 134B, 134C, and 134D that are respectively separated by four longitudinally extending slits 135A, 135B, 135C, and 135D. The illustrated struts 134A, 134B, 134C, and 134D each include an incising element 136, although such is not required. It should be appreciated that the expandable portion 132 may have any number or configuration of struts and incising elements as desired.

As shown in Fig. 8, the illustrated expandable portion 132 further includes recessed portions 160 that respectively extend into the outer surfaces of the struts 134A, 134B, 134C, and 134D. For example, the struts 134A, 134B, 134C, and 134D can be slightly bowed inwardly toward the inner sleeve 140 when in the closed position or, alternatively, may have a reduced thickness along a central portion thereof to create the recessed portions 160. The illustrated incising elements 136 are respectively disposed within the recessed portions 160. Thus, when the catheter tube 130 is inserted into a blood vessel, as described above, the recessed portions 160 help to prevent the incising elements 136 from coming into contact with inner walls of the blood vessel. On the other hand, when the expandable portion 132 is expanded to an opened position, as explained below, the incising elements 136 become exposed from the recessed portions 160. It should be appreciated that the recessed portions 160 can eliminate or reduce the need for the protective sheath (not shown). The guide wire 152 may extend through the entire device.

The expandable portion 132 can be operated between a closed position (shown in Fig. 8) and an opened position (shown in Figs. 9 and 10) by selective movement of the inner sleeve 140 relative to the catheter tube 130, as described above in the first embodiment. Alternatively (or in addition), the struts 134A, 134B, 134C, and 134D can be biased in the opened position. In such an embodiment, the protective sheath (not shown) can be used to effect movement of the expandable portion 132 between the closed position and the opened position.

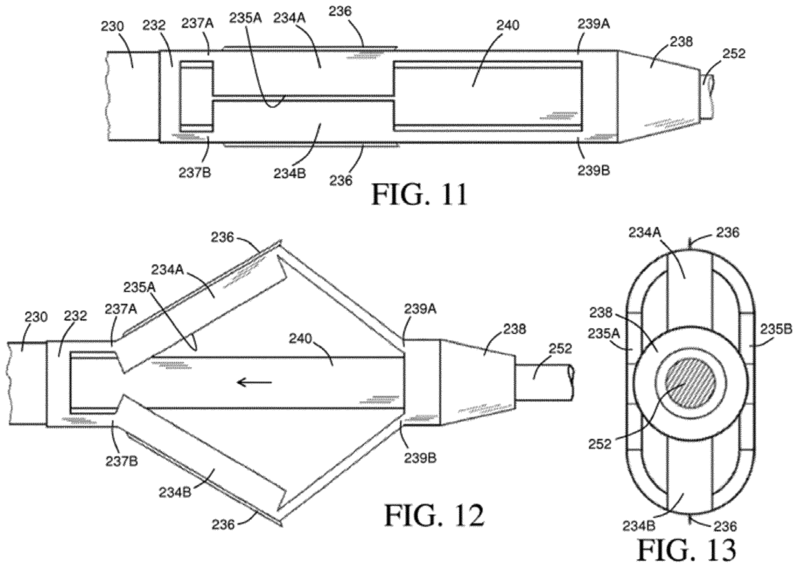

Referring now to Figs. 11 through 13, there is illustrated a catheter tube 230 having an expandable portion 232. The catheter tube 230 and the expandable portion 232 may include any structural features as described and illustrated above in the previous embodiments, although such is not required. Similar features have been numbered with common reference numerals but have been increased by 200 (i.e., 210, 220, 230, etc.). It should be appreciated that similar features are structured similarly, operate similarly, and/or have the same function unless otherwise indicated by the drawings or this specification.

For example, the catheter tube 230 may extend from a handle assembly (not shown) as described above in the first embodiment. The expandable portion 232 is provided on a distal end of the catheter tube 230 and includes a pair of struts 234A and 234B that are separated by a pair of longitudinally extending slits 235A and 235B. The catheter tube 230 may also include a tip member 238, an inner sleeve 240, and a protective sheath (not shown), which is described above in the first embodiment. The guide wire 252 may extend through the entire device.

In the illustrated embodiment, however, the expandable portion 232 includes a first pair of weakened regions 237A, 237B and a second pair of weakened regions 239A, 239B that are respectively located at opposite ends of the struts 234A and 234B. The illustrated weakened regions 237A, 237B and 239A, 239B are formed by enlarged apertures that extend through side walls of the expandable portion 232 that function as hinges. The weakened regions 237A, 237B and 239A, 239B may help reduce the amount of bending stress in the side walls of the expandable portion 232 when the struts 234A and 234B are moved to an opened position. The struts 234A and 234B may include any number or configuration of weakened regions. Further, it should be appreciated that any of the other embodiments in this disclosure may also include weakened regions 237A, 237B and 239A, 239B.

The illustrated struts 234A and 234B remain generally flat along respective lengths thereof in both a closed position (shown in Fig. 11) and an opened position (shown in Figs. 12 and 13) so as to form an apex, although such a configuration is not required. The incising elements 236 are provided along the generally flat portion of the respective struts 234A and 234B. As such, the incising elements 236 may also function as stiffening members for increasing the strength of the struts 234A and 234B. Further, this configuration can reduce the amount of stress in the connection between the incising elements 236 and the struts 234A and 234B, which may otherwise be caused by bowing of the struts 234A and 234B.

As shown in Fig. 12, end portions of the incising elements 236 may extend beyond the apex that is formed by each of the respective struts 234A and 234B. This configuration can increase the effective height of the incising elements 236 when the expandable portion 232 is in the opened position. As such, the incising elements 236 may have a reduced height when the expandable portion 232 is in the closed position, which may eliminate the need for the protective sheath (not shown).

The expandable portion 232 can be operated between the closed position and the opened position by selective movement of the inner sleeve 240 relative to the catheter tube 230, as described above in the first embodiment. Alternatively (or in addition), the struts 234A and 234B can be biased in the opened position. In such an embodiment, the protective sheath (not shown) can be used to effect movement of the expandable portion 232 between the closed position and the opened position.

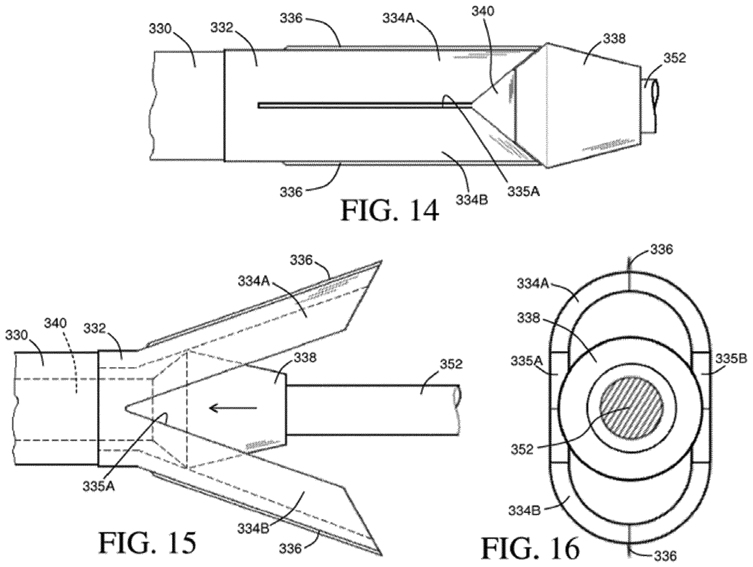

Referring now to Figs. 14 through 16, there is illustrated a catheter tube 330 having an expandable portion 332. The catheter tube 330 and the expandable portion 332 may include any structural features as described and illustrated above in the previous embodiments, although such is not required. Similar features have been numbered with common reference numerals but have been increased by 300 (i.e., 310, 320, 330, etc.). It should be appreciated that similar features are structured similarly, operate similarly, and/or have the same function unless otherwise indicated by the drawings or this specification.

For example, the catheter tube 330 may extend from a handle assembly (not shown) as described above in the first embodiment. The expandable portion 332 is provided on a distal end of the catheter tube 330 and may include a tip member 338. The catheter tube 330 may also include an inner sleeve 340 that is attached to the tip member 338 and a protective sheath (not shown), which is also described above in the first embodiment. The guide wire 352 may extend through the entire device.

In the illustrated embodiment, however, the expandable portion 332 includes a pair of struts 334A and 334B that are supported thereon in a cantilevered manner (i.e., not attached to one another or to the tip member 338 at their distal ends), the purpose of which will be explained below. The struts 334A and 334B are separated by a pair of longitudinally extending slits 335A and 335B that extend from the end of the expandable portion 332. A pair of incising elements 336 is respectively provided along outer surfaces of the struts 334A and 334B. It should be appreciated, however, that the expandable portion 332 may have any number or configuration of struts and incising elements as desired.

As shown in Figs. 15 and 16, the illustrated struts 334A and 334B are supported on the expandable portion 332 so that they can be splayed open in a Y-shaped configuration. For example, the struts 334A and 334B can be splayed open by drawing the inner sleeve 340 within the catheter tube 330, as described above in the first embodiment. In doing so, the tip member 338 slides along the inner surfaces of the struts 334A and 334B and pivots them outwardly. Alternatively (or in addition), the struts 334A and 334B can be biased in the splayed open position. In such an embodiment, the protective sheath (not shown) can be used to effect movement of the expandable portion 332 between a closed position and the splayed open position.

The struts 334A and 334B remain generally flat along their respective lengths in both a closed position (shown in Fig. 14) and the splayed open position, although such is not required. As such, the incising elements 336 may also function as stiffening members for increasing the strength of the struts 334A and 334B. Further, this configuration can reduce the amount of stress in the connection between the incising elements 336 and the struts 334A and 334B, which may otherwise be caused by bowing of the struts 334A and 334B.

As shown in Fig. 15, end portions of the incising elements 336 may extend beyond the distal ends of the respective struts 334A and 334B. This configuration can increase the effective height of the incising elements 336 when the expandable portion 332 is in the splayed open position. As such, the incising elements 336 may have a reduced height when the expandable portion 332 is in the closed position, which may eliminate the need for the protective sheath (not shown).

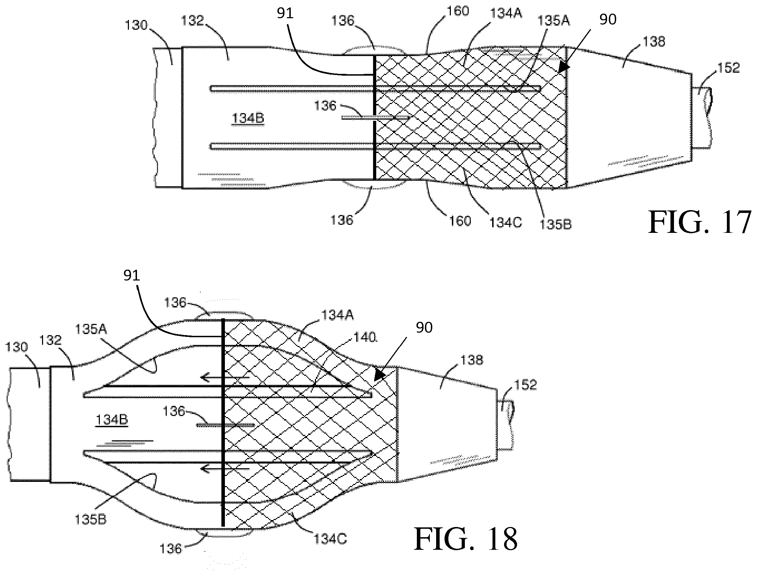

Figs. 17 and 18 illustrate another exemplary disclosure. Specifically, Fig. 17 illustrates the device of Fig. 8 with an exemplary embolic protection device 90 installed thereon, while Fig. 18 illustrates the device of Fig. 17 with the expandable portion 132 in the opened position. The embolic protection device 90 may comprise a basket configured to trap emboli for subsequent removal from the treatment site.

The basket may surround the outer surface of the struts 134A, 134B, and 134C as well as cover the gaps between said struts 134A, 134B, and 134C when the expandable portion 132 is in both the closed and the opened positions. To accomplish this, the basket may be configured to stretch and deform or may comprise excess and/or overlapping material when the expandable portion 132 is in the closed position that unfurls when the expandable portion 132 is in the opened position.

The basket may also cover the front and/or rear of the expandable portion 132. However, in exemplary embodiments the basket may be open on the proximal end and attached, sealed, bonded, or otherwise adhered to the tip member 138 on the distal end. In this way, the basket creates an opening on the proximal end, and is substantially or partially sealed on the distal end by the combination of the basket, guide wire 152, inner sleeve 140, and struts 134A, 134B, and 134C. As will be explained in greater detail herein, the basket may be comprised of a woven material or otherwise comprise a plurality of apertures along the side walls thereof. In exemplary embodiments these apertures may be configured to permit blood to flow therethrough while preventing emboli and other particulate over a specified size from traveling therebeyond. In this way, the blood flow, and any emboli or other particulate contained therein, are forced to enter the basket's opening on the proximal end and leave only if the matter fits through the apertures provided in the sidewalls of the basket.

In exemplary embodiments the basket is comprised of a mesh. The mesh may be of any size, shape, or configuration. The mesh may be comprised of nitinol, polytetrafluoroethylene (PTFE), a metallic, a polymer, or the like and may extend over any section or the entirety of the expandable portion 132. The mesh may be woven such that the apertures are sized to permit blood (including, for example without limitation, healthy cells, plasma, and platelets) flow therethrough, while trapping emboli and other particulate larger than the apertures provided in the mesh.

The embolic protection device 90 may extend over some or all of the expandable portion 132. In exemplary embodiments the embolic protection device 90 extends over substantially the distal half of the expandable portion 132 while the incising elements 136 may extend over substantially the proximal half of the expandable portion 132. Regardless, an expandable hoop 91 may be located on the outer surface of the expandable potion 132 and may assist in securing and preserving the shape of the basket. The expandable hoop 91 may be located at substantially the midline of the expandable portion 132, though any location is contemplated. The expandable hoop 91 may be comprised of nitinol, PTFE, a metallic, a polymer, or the like and may be configured to expand and collapse when the expandable portion is moved between the opened and the closed positions.

The expandable hoop 91 may be configured to match the outer diameter of the expandable portion 132 when the expandable portion 132 is in both the opened and the closed positions and may be configured to fit inside the sheath 42 if one is being used. In exemplary embodiments the expandable hoop 91 may operate by a telescoping mechanism such that portions of the expandable hoop 91 slide atop one another. In other exemplary embodiments, the expandable hoop 91 may be configured to deform by elongating and reorienting at an increased lateral angle such that the expandable hoop 91 may be placed substantially flush with the outer surface of the expandable portion 132, when the expandable portion 132 is in the collapsed position and/or is forced inside the sheath 42.

The expandable hoop 91 may provide an attachment point for the basket. For example, but not to serve as a limitation, in embodiments where the basket is the mesh or other woven material, the mesh may be woven around the expandable hoop 91. In other exemplary embodiments, the basket may be welded, soldered, adhered, or otherwise bonded to the expandable hoop 91 and/or directly to the struts 134A, 134B, and 134C or other parts of the intravascular catheter device 10.

In exemplary methods the expandable portion 132 may be placed in the closed position and positioned downstream from the treatment area. The expandable portion 132 may be placed in the opened position. The embolic protection device 90 may be automatically deployed when the expandable portion 132 is placed in the opened position as the basket, and the optional expandable hoop 91 if utilized, may expand when the struts 134A, 134B, and 134C are placed in the opened position and collapse when the struts 134A, 134B, and 134C are placed in the closed position. However, in exemplary embodiments the expandable hoop 91 may be moved between the closed and the opened positions independently of the expandable portion 132. For example, but without limitation, the expandable hoop 91 may be configured to automatically expand when removed from the sheath 42. Regardless, the expandable portion 132 may next be retracted along some or all of the treatment area to facilitate fragmentation of the atherosclerotic plaque. The expandable portion 132 and the embolic protection device 90 may then be placed in the closed position, thereby trapping any emboli caught in the basket during the procedure. This process may be repeated multiple times over the same treatment area or over multiple treatment areas.

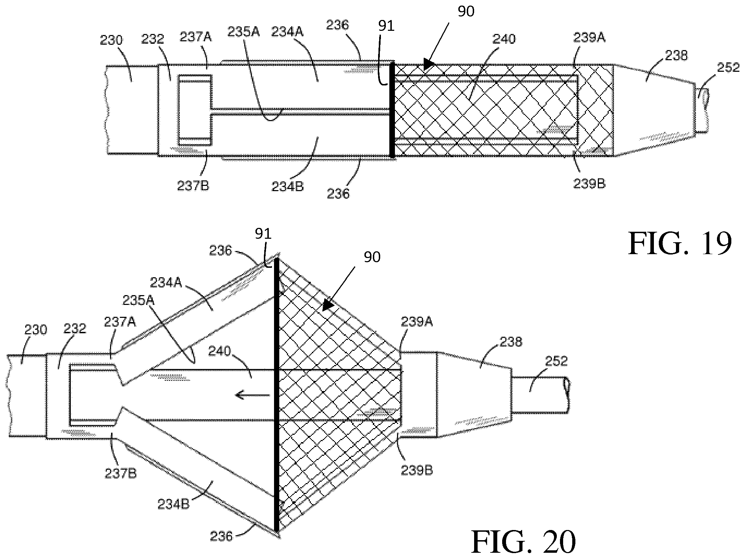

Similarly, Fig. 19 illustrates the device of Fig. 11 with another exemplary embolic protection device 90 installed thereon, while Fig. 20 illustrates the device of Fig. 19 with the expandable portion 232 in the opened position. The embolic protection device 90 may be similar to the one shown and described with respect to Figs. 17 and 18, and may be similarly operated.

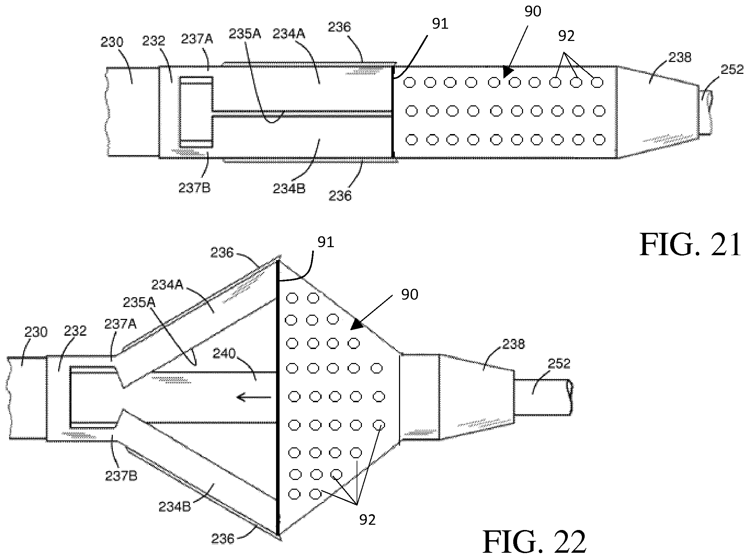

Figs. 21 and 22 illustrate another exemplary embodiment of the embolic protection device 90. The basket of the embolic protection device 90 may be a film or covering having a number of apertures 92 located therein. Any size, shape, number, and configuration of the apertures 92 are contemplated. In exemplary disclosures the film or covering is comprised of PTFE, though any material is contemplated. Similarly, the film or covering may be attached to the expandable hoop 91, though such is not required. The embolic protection device 90 may otherwise be similar to the one shown and described with respect to Figs. 17-20, and may be similarly operated.

It is notable that while the embolic protection device 90 is illustrated with respect to the devices of Figs. 8-9 and 11-12, it is contemplated that the embolic device 90 may be utilized with any of the disclosures as shown and/or described herein.

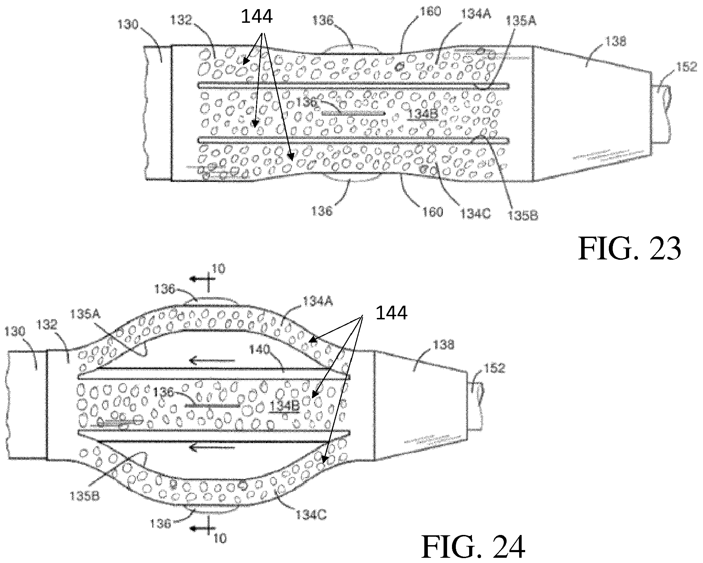

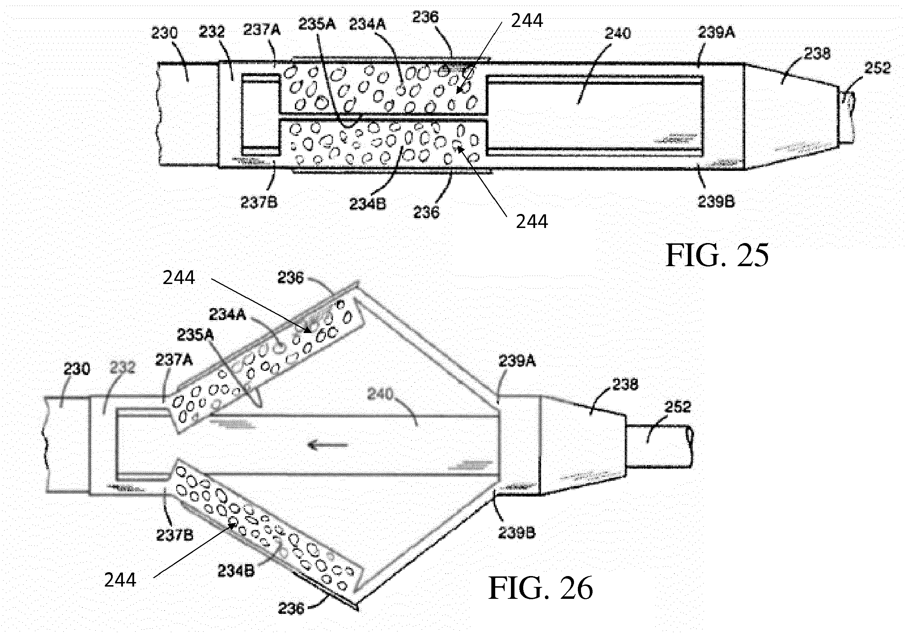

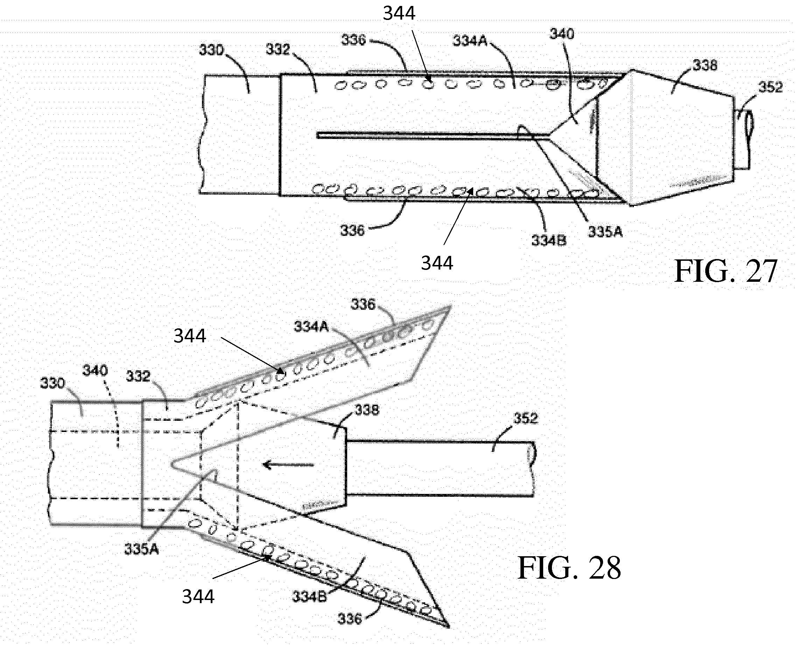

Fig. 23 through Fig. 30 illustrate a grating tool 144, 244, and 344 located on the expandable portion 132, 232, and 332, respectively. The grating tool 144, 244, and 344 may be placed upon any number of the struts 134A, 134B, and 134C (or 234A and 234B or 334A and 334B). The grating tool 144, 244, and 344 may extend along the entire strut, an upper, lower, distal, proximal, or other portion thereof. The grating tool 144, 244, and 344 may be any kind of device configured to grate, shred, peel, file, shave, cut, or remove any structure or obstruction in the blood vessel 50. In exemplary embodiments, the grating tool 144, 244, and 344 may be configured to perform atherectomy on atherosclerotic material 54 located in the blood vessel 50 by moving the expandable portion 132, 232, and 332 axially through the blood vessel 50. However, the grating tool 144, 244, and 344 may be configured specifically for any particular purpose.

In exemplary embodiments, the grating tool 144, 244, and 344 comprises a number of grating apertures located along the struts 134A, 134B, and 134C (or 234A and 234B or 334A and 334B). The grating apertures may be similar to those used on a cheese grater or microplane, but sized and configured to fit on the struts 134A, 134B, and 134C (or 234A and 234B or 334A and 334B) and grate a substance or obstruction located within the blood vessel 50. In exemplary embodiments, the substance or obstruction to be grated is atherosclerotic material 54. The grating apertures may contain a first portion that is elevated or depressed relative to a second portion such that the substance or obstruction is removed when the grating tool 144, 244, and 344 is passed over the surface of the substance or obstruction. For example, but not to serve as a limitation, the elevated or depressed portion may be an edge of the grating aperture or may be a tab or other protrusion into the grating aperture. In addition, or alternatively, the grating apertures may comprise a sharpened or roughened portion that is configured to remove atherosclerotic material 54 when the grating tool 144, 244, and 344 is passed over the surface thereof.

The grating apertures may be of any size or shape. For example, but not to serve as a limitation, the grating apertures may be circular, oval, square, rectangular, or the like. Further, the grating apertures may be spaced apart along the struts 134A, 134B, and 134C (or 234A and 234B or 334A and 334B) randomly or in any pattern. In exemplary embodiments, the grating apertures are formed by punching, stamping, drilling, or otherwise cutting them out of the otherwise solid struts 134A, 134B, and 134C (or 234A and 234B or 334A and 334B). However, in other exemplary embodiments, the grating apertures may be integrally formed with the struts.

The grating tool 144, 244, and 344 may be configured such that grating is performed when the expandable portion 132, 232, and 332 is moved axially through the blood vessel 50 forwards or backwards. In exemplary embodiments, this may be accomplished by alternating or otherwise varying the location of the relatively elevated surface (i.e., the first portion). Additionally, or in the alternative, this may be accomplished by alternating or otherwise varying the location of the sharpened or roughened portion on the distal or proximal side of the grating aperture as appropriate.

In other exemplary embodiments, the grating tool 144, 244, and 344 may be configured such that grating is performed only when the expandable portion 132, 232, and 332 is moved axially through the blood vessel 50 in a particular direction. For example, but not to serve as a limitation, the grating tool 144, 244, and 344 may be configured to grate the atherosclerotic material 54 when the expandable portion 132, 232, and 332 is retracted axially through the blood vessel 50, but is configured to not grate the atherosclerotic material 54 when the expandable portion 132, 232, and 332 is advanced axially through the blood vessel 50. Similarly, the grating tool 144, 244, and 344 may be configured to grate the atherosclerotic material 54 when the expandable portion 132, 232, and 332 is advanced axially through the blood vessel 50, but is configured to not grate the atherosclerotic material when the expandable portion is retracted axially through the blood vessel 50.

In exemplary embodiments, this may be accomplished by providing the relatively elevated surface (i.e., the first portion) on the distal or proximal side of the grating aperture as appropriate. Additionally, or in the alternative, this may be accomplished by providing the sharpened or roughened portion on the distal or proximal side of the grating aperture as appropriate. For example, but not to serve as a limitation, to grate the atherosclerotic material 54 when the expandable portion 132, 232, and 332 is retracted axially through the blood vessel 50, the relatively higher surface (i.e., the first portion) and/or the sharpened or roughened portion may be provided on the distal portion of the apertures.

The grating tool 144, 244, and 344 may be located such that it contacts the substance or obstruction to be grated when the expandable portion 132, 232, and 332 is placed in the opened position. The grating tool 144, 244, and 344 may be configured such that it does not contact the blood vessel 50 wall or the structure or obstruction to be grated when the expandable portion 132, 232, and 332 is in the closed position. Regardless, a protective sheath 42 may be utilized which is configured to substantially surround and protect the grating tool 144, 244, and 344 from contact with surrounding blood vessel 50 or structures or obstructions therein when the expandable portion 132, 232, and 332 is in the closed position. The expandable portion 132, 232, and 332 may be advanced to a zone of attention within the blood vessel 50 and moved into the opened position such that the grating tool 144, 244, and 344 contacts the surface of the atherosclerotic material 54. The expandable portion, 132, 232, and 332 may then by moved axially through the blood vessel 50 to perform atherectomy.

The grating tool 144, 244, and 344 may be used with or without the incising elements 136, 236, and 336. In such embodiments, the incising elements 136, 236, and 336 may extend into the atherosclerotic material 54, scoring it and facilitating fragmentation of the atherosclerotic material 54. Simultaneously, the grating tool 144, 244, and 344 may ride along the surface of the atherosclerotic material 54, grating it and facilitating its removal.

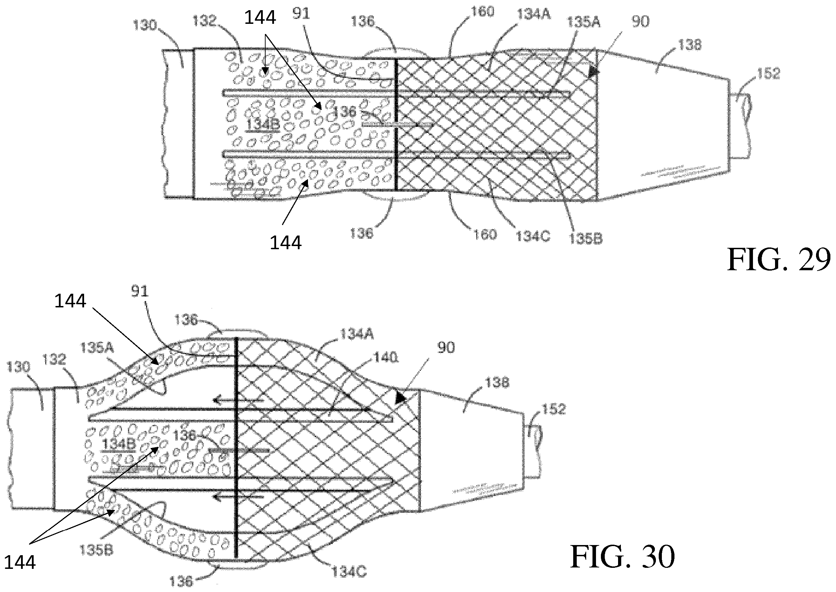

It is contemplated that the grating tool 144, 244, and 344 may be used with any of the embodiments shown or described herein. According to the invention as illustrated in Fig. 29 and Fig. 30, the grating tool 144, 244, and 344 is used in combination with the embolic protection device 90, with the incising elements 136, 236, and 336. In this way, the atherosclerotic material 54 grated or otherwise removed by the grating tool 144, 244, and 344 may be captured by the embolic protection device 90 and removed from the blood vessel 50. In exemplary embodiments, the embolic protection device 90 may be sized and configured to capture the atherosclerotic material 54 fragments that would be created based on the size and type of the grating apertures or other grating tool 144, 244, and 344 used.

The use of the embolic protection device 90 with the grating tool 144, 244, and 344 is not limited to the embodiment shown in Fig. 29 and Fig. 30. It is contemplated that the grating tool 144, 244, and 344, the embolic protection device 90, and/or the incising elements 136, 236, and 336 may be used with any of the embodiments shown and described herein. While the grating tool 144, 244, and 344 is often described as being used to grate or otherwise remove atherosclerotic material 54, those having skill in the arts will recognize that the grating tool 144, 244, and 344 may be used to grate or otherwise remove any structure or obstruction located in the blood vessel 50.

The principle and mode of operation of this invention have been explained and illustrated in its preferred embodiments.

## Claims

1. An intravascular catheter device (10) for treating a zone of attention located within a blood vessel (50) comprising atherosclerotic material (54), said device (10) comprising:
a flexible catheter tube (30, 130, 230, 330);
a handle assembly (20), wherein the flexible catheter tube (30, 130, 230, 330) extends from the handle assembly (20);
an expandable portion (32, 132, 232, 332) secured to a distal end of said catheter tube (30, 130, 230, 330) and comprising a plurality of struts (34A, 34B, 134A, 134B, 134C, 134D, 234A, 234B, 334A, 334B) each defining an outward facing surface, the expandable portion (32, 132, 232, 332) being operable between a closed position, wherein the expandable portion (32, 132, 232, 332) has a first diameter, and an opened position, wherein the expandable portion (32, 132, 232, 332) has a second diameter that is larger than the first diameter;
an inner sleeve (40, 140, 240, 340) traveling through the flexible catheter tube (30, 130, 230, 330) and in communication with the handle assembly (20), wherein the inner sleeve (40, 140, 240, 340) is configured to move the expandable portion (32, 132, 232, 332) between the opened and the closed positions;
a grating tool (144, 244, 344) located on at least one of the struts (34A, 34B, 134A, 134B, 134C, 134D, 234A, 234B, 334A, 334B), wherein said grating tool (144, 244, 344) comprises a number of grating apertures and is configured to remove atherosclerotic material (54) when the expandable portion (32, 132, 232, 332) is placed in the opened position and moved axially through the blood vessel (50);
an embolic protection device (90) located on and configured to surround at least a portion of the outer surface of the expandable portion (32, 132, 232, 332) when the expandable portion (32, 132, 232, 332) is located in both the opened and the closed position, wherein the embolic protection device (90) is configured to capture the fragments of the atherosclerotic material (54) grated by the grating tool (144, 244, 344); and
an incising member (36, 136, 236, 336) provided on and extending from the outward facing surface of at least one of the struts (34A, 34B, 134A, 134B, 134C, 134D, 234A, 234B, 334A, 334B), the incising member (36, 136, 236, 336) having a sharpened edge located along an upper edge thereof wherein said sharpened edge extends substantially in parallel with a longitudinal axis of the expandable portion (32, 132, 232, 332) when the expandable portion (32, 132, 232, 332) is in the closed position and is configured to score atherosclerotic material (54) when moved axially through in the blood vessel (50).

2. The intravascular catheter device (10) of claim 1 wherein:
each grating aperture comprises a first portion that is elevated relative to a second portion thereof.

3. The intravascular catheter device (10) of claims 1 or 2 wherein:
the grating apertures comprise a sharpened or roughened portion.

4. The intravascular catheter device (10) of any of claims 1-3 wherein:
the grating apertures extend over substantially the proximal half of the struts (34A, 34B, 134A, 134B, 134C, 134D, 234A, 234B, 334A, 334B).

5. The intravascular catheter device of any of claims 1-4 wherein:
the grating apertures extend over the majority of the struts (34A, 34B, 134A, 134B, 134C, 134D, 234A, 234B, 334A, 334B).

6. The intravascular catheter device (10) of claim 1 wherein:
the grating tool (144, 244, 344) is located on a distal portion of the expandable portion (32, 132, 232, 332); and
the embolic protection device (90) is located on a proximal portion of the expandable portion (32, 132, 232, 332).

7. The intravascular catheter device (10) of any of claims 1-6 further comprising:
a protective sheath (42) configured to selectively surround the expandable portion (32, 132, 232, 332) and the grating tool (144, 244, 344) when the expandable portion (32, 132, 232, 332) is in the closed position.

8. The intravascular catheter device (10) of claim 1 further comprising:
a tip member (38, 138, 238, 338) connected to a distal end of the inner sleeve (40, 140, 240, 340) and configured to move the expandable portion (32, 132, 232, 332) between the opened and the closed position.

## Patentansprüche

1. Intravaskuläre Kathetervorrichtung (10) zur Behandlung eines Bereichs von Interesse innerhalb eines Blutgefäßes (50), umfassend atherosklerotisches Material (54), wobei die Vorrichtung (10) folgendes umfasst:
einen flexiblen Katheterschlauch (30, 130, 230, 330);
eine Griffanordnung (20), wobei der flexible Katheterschlauch (30, 130, 230, 330) sich von der Griffanordnung (20) erstreckt;
einen expandierbaren Abschnitt (32, 132, 232, 332,), der an einem distalen Ende des Katheterschlauchs (30, 130, 230, 330) befestigt ist und mehrere Streben (34A, 34B, 134A, 134B, 134C, 134D, 234A, 234B, 334A, 334B) umfasst, von denen jede eine nach außen gerichtete Fläche definiert, wobei der expandierbare Abschnitt (32, 132, 232, 332) zwischen einer geschlossenen Position, in welcher der expandierbare Abschnitt (32, 132, 232, 332) einen ersten Durchmesser aufweist, und einer geöffneten Position, in welcher der expandierbare Abschnitt (32, 132, 232, 332) einen zweiten Durchmesser aufweist, der größer ist als der erste Durchmesser, betreibbar ist;
eine Innenhülse (40, 140, 240, 340), die sich durch den flexiblen Katheterschlauch (30, 130, 230, 330) bewegt und mit der Griffanordnung (20) in Kontakt steht, wobei die Innenhülse (40, 140, 240, 340) dazu ausgelegt ist, den expandierbaren Abschnitt (32, 132, 232, 332) zwischen der geöffneten und geschlossenen Position zu bewegen;
ein Reibeinstrument (144, 244, 344), das an mindestens einer der Streben (34A, 34B, 134A, 134B, 134C, 134D, 234A, 234B, 334A, 334B) angeordnet ist, wobei das Reibeinstrument (144, 244, 344) eine Reihe an Reibeöffnungen umfasst und dazu ausgelegt ist, atherosklerotisches Material (54) zu entfernen, wenn der expandierbare Abschnitt (32, 132, 232, 332) sich in der geöffneten Position befindet und axial durch das Blutgefäß (50) bewegt wird;
eine Embolieschutzvorrichtung (90), die an der Außenfläche des expandierbaren Abschnitts (32, 132, 232, 332) angeordnet und dazu ausgelegt ist, mindestens einen Abschnitt davon zu umgeben, wenn der expandierbare Abschnitt (32, 132, 232, 332) sich sowohl in der geöffneten als auch in der geschlossenen Position befindet, wobei die Embolieschutzvorrichtung (90) dazu ausgelegt ist, die Fragmente des atherosklerotischen Materials (54), die von dem Reibeinstrument (144, 244, 344) abgerieben wurden, abzufangen; und
ein Einschneideelement (36, 136, 236, 336), das an der nach außen gerichteten Fläche von mindestens einer der Streben (34A, 34B, 134A, 134B, 134C, 134D, 234A, 234B, 334A, 334B) angeordnet ist und sich davon erstreckt, wobei das Einschneideelement (36, 136, 236, 336) entlang einer oberen Kante davon eine scharfe Kante aufweist, wobei die scharfe Kante sich im Wesentlichen parallel entlang einer Längsachse des expandierbaren Abschnitts (32, 132, 232, 332) erstreckt, wenn der expandierbare Abschnitt (32, 132, 232, 332) sich in der geschlossenen Position befindet, und die dazu ausgelegt ist, atherosklerotisches Material (54) zu erbeuten, wenn sie axial durch das Blutgefäß (50) bewegt wird.

2. Intravaskuläre Kathetervorrichtung (10) nach Anspruch 1, wobei:
jede Reibeöffnung einen ersten Abschnitt umfasst, der relativ zu einem zweiten Abschnitt davon erhöht ist.

3. Intravaskuläre Kathetervorrichtung (10) nach Anspruch 1 oder 2, wobei:
die Reibeöffnungen einen geschärften oder gerauten Abschnitt umfassen.

4. Intravaskuläre Kathetervorrichtung (10) nach einem der Ansprüche 1 bis 3, wobei:
die Reibeöffnungen sich im Wesentlichen über die proximale Hälfte der Streben (34A, 34B, 134A, 134B, 134C, 134D, 234A, 234B, 334A, 334B) erstrecken.

5. Intravaskuläre Kathetervorrichtung nach einem der Ansprüche 1 bis 4, wobei:
die Reibeöffnungen sich über den Großteil der Streben (34A, 34B, 134A, 134B, 134C, 134D, 234A, 234B, 334A, 334B) erstrecken.

6. Intravaskuläre Kathetervorrichtung (10) nach Anspruch 1, wobei:
das Reibeinstrument (144, 244, 344) an einem distalen Abschnitt des expandierbaren Abschnitts (32, 132, 232, 332) angeordnet ist; und
die Embolieschutzvorrichtung (90) an einem proximalen Abschnitt des expandierbaren Abschnitts (32, 132, 232, 332) angeordnet ist.

7. Intravaskuläre Kathetervorrichtung (10) nach einem der Ansprüche 1 bis 6, weiterhin umfassend:
eine Schutzhülle (42), die dazu ausgelegt ist, den expandierbaren Abschnitt (32, 132, 232, 332) und das Reibeinstrument (144, 244, 344) selektiv zu umgeben, wenn der expandierbare Abschnitt (32, 132, 232, 332) sich in der geschlossenen Position befindet.

8. Intravaskuläre Kathetervorrichtung (10) nach Anspruch 1, weiterhin umfassend:
ein Spitzenelement (38, 138, 238, 338), das mit einem distalen Ende der Innenhülse (40, 140, 240, 340) verbunden und dazu ausgelegt ist, den expandierbaren Abschnitt (32, 132, 232, 332) zwischen der geöffneten und der geschlossenen Position zu bewegen.

## Revendications

1. Un dispositif de cathéter intravasculaire (10) pour traiter une zone d'attention située dans un vaisseau sanguin (50) comprenant la matière athéroscléreuse (54), ledit dispositif (10) comprenant :
un tube de cathéter flexible (30, 130, 230, 330) ;
un ensemble poignée (20), dans lequel le tube de cathéter flexible (30, 130, 230, 330) s'étend à partir de l'ensemble poignée (20) ;
une partie extensible (32, 132, 232, 332) fixée à une extrémité distale dudit tube de cathéter (30, 130, 230, 330) et comprenant une pluralité d'entretoises (34A, 34B, 134A, 134B, 134C, 134D, 234A, 234B, 334A, 334B) définissant chacune une surface tournée vers l'extérieur, la partie extensible (32, 132, 232, 332) pouvant être actionnée entre une position fermée, dans lequel la partie extensible (32, 132, 232, 332) a un premier diamètre, et une position ouverte, dans lequel la partie extensible (32, 132, 232, 332) a un deuxième diamètre qui est plus grand que le premier diamètre ;
un manchon intérieur (40, 140, 240, 340) se déplaçant à travers le tube de cathéter flexible (30, 130, 230, 330) et en communication avec l'ensemble poignée (20), dans lequel le manchon intérieur (40, 140, 240, 340) est configuré pour déplacer la partie extensible (32, 132, 232, 332) entre les positions ouverte et fermée ;
un outil de râpage (144, 244, 344) situé sur au moins une des entretoises (34A, 34B, 134A, 134B, 134C, 134D, 234A, 234B, 334A, 334B), dans lequel ledit outil de râpage (144, 244, 344) comprend un certain nombre d'ouvertures de râpage et est configuré pour enlever la matière athéroscléreuse (54) lorsque la partie extensible (32, 132, 232, 332) est placée en position ouverte et déplacée axialement à travers le vaisseau sanguin (50) ;
un dispositif de protection embolique (90) situé sur et configuré pour entourer au moins une partie de la surface extérieure de la partie extensible (32, 132, 232, 332) lorsque la partie extensible (32, 132, 232, 332) est située à la fois en position ouverte et en position fermée, dans lequel le dispositif de protection embolique (90) est configuré pour capturer les fragments de la matière athéroscléreuse (54) râpée par l'outil de râpage (144, 244, 344) ; et
un élément d'incision (36, 136, 236, 336) prévu sur et s'étendant à partir de la surface tournée vers l'extérieur d'au moins une des entretoises (34A, 34B, 134A, 134B, 134C, 134D, 234A, 234B, 334A, 334B), l'élément d'incision (36, 136, 236, 336) ayant un bord aiguisé situé le long d'un bord supérieur de celui-ci, dans lequel ledit bord aiguisé s'étend sensiblement parallèlement à un axe longitudinal de la partie extensible (32, 132, 232, 332) lorsque la partie extensible (32, 132, 232, 332) est dans la position fermée et est configurée pour marquer la matière athéroscléreuse (54) lorsqu'elle est déplacée axialement à travers le vaisseau sanguin (50).

2. Le dispositif de cathéter intravasculaire (10) selon la revendication 1 dans lequel :
chaque ouverture de râpage comprend une première partie qui est élevée par rapport à une deuxième partie de celle-ci.

3. Le dispositif de cathéter intravasculaire (10) selon la revendication 1 ou 2 dans lequel :
les ouvertures de râpage comportent une partie aiguisée ou rugueuse.

4. Le dispositif de cathéter intravasculaire (10) selon l'une quelconque des revendications 1 à 3 dans lequel :
les ouvertures de râpage s'étendent sensiblement sur la moitié proximale des entretoises (34A, 34B, 134A, 134B, 134C, 134D, 234A, 234B, 334A, 334B).

5. Le dispositif de cathéter intravasculaire selon l'une quelconque des revendications 1 à 4 dans lequel :
les ouvertures de râpage s'étendent sur la majorité des entretoises (34A, 34B, 134A, 134B, 134C, 134D, 234A, 234B, 334A, 334B).

6. Le dispositif de cathéter intravasculaire (10) selon la revendication 1 dans lequel :
l'outil de râpage (144, 244, 344) est situé sur une partie distale de la partie extensible (32, 132, 232, 332) ; et
le dispositif de protection embolique (90) est situé sur une partie proximale de la partie extensible (32, 132, 232, 332).

7. Le dispositif de cathéter intravasculaire (10) selon l'une quelconque des revendications 1 à 6 comprenant en outre :
une gaine de protection (42) configurée pour entourer sélectivement la partie extensible (32, 132, 232, 332) et l'outil de râpage (144, 244, 344) lorsque la partie extensible (32, 132, 232, 332) est dans la position fermée.

8. Le dispositif de cathéter intravasculaire (10) selon la revendication 1 comprenant en outre :
un élément de pointe (38, 138, 238, 338) relié à une extrémité distale du manchon intérieur (40, 140, 240, 340) et configuré pour déplacer la partie extensible (32, 132, 232, 332) entre la position ouverte et la position fermée.
